# EUROPÄISCHE PATENTSCHRIFT

(11) **EP 0 360 277 B1**
(45) Veröffentlichungstag und Bekanntmachung des Hinweises auf die Patenterteilung: **08.06.1994**
(21) Anmeldenummer: 89117522.6
(22) Anmeldetag: 22.09.1989
(51) Int. Cl.: A61F 13/08, A61F 13/00

(54) **Aus elastischem Textilmaterial bestehende Bandage**
Bandage consisting of elastic textile material
Bandage consistant en matière textile élastique

(30) Priorität: 23.09.1988 DE 3832438
(43) Veröffentlichungstag der Anmeldung: 28.03.1990
(73) Patentinhaber: Bauerfeind GmbH & Co., D-47880 Kempen (DE)
(72) Erfinder: Reinhardt, Holger, D-4152 Kempen 1 (DE)
(74) Vertreter: Bardehle, Heinz, Dipl.-Ing.

(56) Entgegenhaltungen:
- EP-A- 0 242 766
- US-A- 3 799 167
- US-A- 4 116 236
- US-A- 4 201 203

## Beschreibung

Die Erfindung bezieht sich auf eine aus elastischem Textilmaterial bestehende Bandage zur Behandlung von Gelenken, z.B. einen Strumpf, gemäß dem Oberbegriff des Patentanspruchs 1.

Derartige Bandagen sind beispielswiese aus der US-A-4 116 236 bekannt und werden zur Behandlung von Gelenken verwendet, z.B. des Knie- oder Sprunggelenkes, wobei zur Vermeidung von Druckstellen die Bandage mit Polstern versehen ist. Bisher hat man diese Polster an dem Grundmaterial der Bandage, üblicherweise ein elastisches, gestricktes Textilmaterial durch Aufnähen eines ebenfalls aus einem derartigen Textilmaterial bestehenden Überzuges befestigt. Der Überzug bildet dabei um die Bandage einen Rand, der, das Polster umschließend, an dem Grundmaterial festgenäht ist. Um dabei die Elastizität der Bandage nicht zu beeinträchtigen, werden dabei Stichmuster,z.B. Zick-Zack-Stich, angewendet, die innerhalb dieser Randnaht dieser eine gewisse Elastizität erhalten. Es ist jedoch eine bekannte Tatsache, daß durch derartige Nähte die Bandage in dem betreffenden Bereich an Elastizität verliert, was natürlich unerwünscht ist.

Es ist bekannt, Polsterkörper, z.B. für Automobilsitze, unter Überzügen anzubringen. Der Polsterkörper wird hierzu auf ein Grundmaterial aufgelegt und von einem Überzug abgedeckt, der dann mit dem Grundmaterial unter Anwendung von Hochfrequenz verschweißt wird. Bei dem Grundmaterial und dem Überzug kann es sich um mit einer Plastikschicht überzogenes Textilmaterial handeln. Die Verschweißung erfolgt dann im Bereich der Berührungskontaktstellen der Plastikbeschichtung.

Eine derartige Befestigungsmethode ist für die hier in Rede stehende Bandage darum ungeeignet, weil sich im Bereich der Schweißnähte eine ausgeprägte Elastizitätsverminderung ergibt, sofern überhaupt die Plastikbeschichtung eine Elastizität zuläßt.

Der Erfindung liegt die Aufgabe zugrunde, die Befestigung des Polsters an der Bandage zu verbessern und diese so zu gestalten, daß dabei im Befestigungsbereich praktisch kein Elastizitätsverlust entsteht. Erfindungsgemäß geschieht dies durch die im Kennzeichen des Patentanspruchs 1 angegebenen Merkmale.

Durch die Anbringung der elastischen, thermoplastischen Kunststoffbeschichtung auf der dem Polster zugewandten Seite des Überzuges behält dieser seine Elastizität im vollen Umfange bei, so daß sich durch die Kunststoffbeschichtung die Dehnbarkeit des Überzuges nicht ändert. Das Gleiche gilt für den Bereich der Verklebung mit dem Textilmaterial der Bandage, da sich bei der Erhitzung des Kunststoffs bis zu seiner Erweichung und der nachträglichen Wiederverfestigung dessen Elastizitätseigenschaften nicht ändern. Die Verklebung mit dem Textilmaterial der Bandage führt dabei nur zu einer oberflächlichen Verbindung mit den Fäden des Textilmaterials, ohne daß diese dabei miteinander verschweißen, wobei sie weitgehend ihre Elastizität verlieren würden. Eine Erweichung der Textilmaterialien wird dabei dadurch vermieden, daß für die Beschichtung des Überzuges ein Kunststoff verwendet wird, dessen Erweichungspunkt unter demjenigen der Textilmaterialien liegt.

Die Erhitzung der Kunststoffbeschichtung nimmt man zweckmäßig in bekannter Weise durch Anwendung von Hochfrequenz vor, wozu das Textilmaterial mit der Bandage mit darüber befindlichem Polster und Überzug auf eine als Platte ausgebildete Hochfrequenzelektrode aufgelegt wird, auf die dann eine den Konturen der gewünschten Verklebungsstrecke folgende zweite Hochfrequenzelektrode aufgesetzt wird. Die dazwischenliegenden Materialien, nämlich das Textilmaterial der Bandage und der Überzug der Kunststoffbeschichtung wirken dabei als Isolator, in dem in bekannter Weise aufgrund der Anwendung der Hochfrequenz die gewünschte Erhitzung eintritt.

Die vorstehend beschriebene Gestaltung der Bandage hat darüber hinaus den Vorteil, daß sie gegenüber dem bisher üblichen Annähen des Überzuges einen wesentlichen Fabrikationsvorteil erbringt, da das Verkleben durch Erhitzung einen einfachen und leicht zu automatisierenden Fabrikationsschritt darstellt.

Als Kunststoffbeschichtung verwendet man zweckmäßig Polyurethan, das bekanntlich gute Elastizitätseigenschaften aufweist.

Man kann die Kunststoffbeschichtung auf den Überzug aufstreichen, wozu der Kunststoff zunächst in einem Lösungsmittel gelöst wird, das sich nach dem Aufstreichen verflüchtigt. Es ist aber auch möglich, die Kunststoffbeschichtung auf den Überzug aufzusprühen, wobei vor allem Bereiche, die später in Kontakt mit dem Polster geraten, z.B. durch Auflegen einer Schablone ausgelassen werden können. In diesem Falle behindert der Kunststoff eine Luft- und Feuchtigkeitsdurchlässigkeit des Überzuges nicht, worauf es vor allem bei grösseren Polstern unter Umständen ankommen kann.

Es ist natürlich auch jede andere bekannte Methode des Aufbringens der Kunststoffbeschichtung auf den Überzug anwendbar.

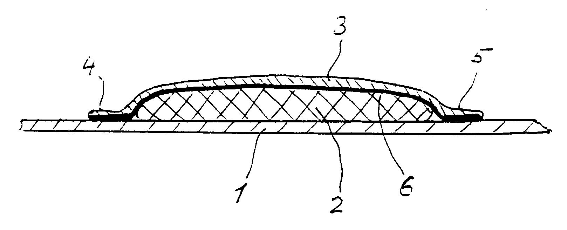

In der Figur ist ein Ausführungsbeispiel der Erfindung dargestellt. Sie zeigt im Schnitt einen Teil einer Bandage mit dem Textilgrundmaterial 1, z.B. ein gestricktes Textilmaterial, auf das das Polster 2 aufgelegt ist. Das Polster 2 kann z.B. aus einem elastischen Schaum bestehen. Das Polster 2 wird von dem Überzug 3 überdeckt, der nur mit seinen Randbereichen 4 und 5 über das Polster 2 hinausragt. Auf seiner dem Polster 2 zugewandten Seite ist der Überzug 3 mit der durch die stärkere Linie bezeichneten Kunststoffbeschichtung 6 versehen, die in den Randbereichen 4 und 5 aufgrund von Erhitzung in diesen Bereichen sich mit der Oberfläche des Textilmaterials 1 verklebt hat. Die die Verklebung aufweisenden Randbereiche 4 und 5 umschließen vollständig oder im wesentlichen vollständig das Polster 2, so daß dieses sicher an dem Textilgrundmaterial 1 befestigt ist. Die Bereiche der Verklebung in den Randbereichen 4 und 5 sind durch die dort dickere Linienführung angedeutet.

## Patentansprüche

1. Aus elastischem Textilmaterial bestehende Bandage (1) zur Behandlung von Gelenken, z.B. Strumpf, die mit einem Polster (2) versehen ist, das von einem Überzug (3) aus gleichem oder ähnlichem Textilmaterial abgedeckt und mittels über das Polster (2) überstehender Ränder an dem Textilmaterial der Bandage (1) befestigt ist, dadurch **gekennzeichnet,** daß der Überzug (3) auf seiner dem Polster (2) zugewandten Seite mit einer elastischen, thermoplastischen Kunststoffbeschichtung (6) versehen ist, die im Bereich der Ränder (4,5) durch Erhitzung mit dem Textilmaterial (1) der Bandage verklebt ist und deren Erweichungstemperatur unter derjenigen der Textilmaterialien liegt.

2. Bandage nach Anspruch 1, dadurch gekennzeichnet, daß die Kunststoffbeschichtung (6) aus Polyurethan besteht.

3. Bandage nach Anspruch 1 oder 2, dadurch gekennzeichnet, daß die Kunststoffbeschichtung (6) auf den Überzug (3) aufgestrichen ist.

4. Bandage nach Anspruch 1 oder 2, dadurch gekennzeichnet, daß die Kunststoffbeschichtung (6) auf den Überzug (3) aufgesprüht ist.

## Claims

1. Bandage (1) consisting of elastic textile material, e.g. a stocking, for the treatment of joints, provided with a pad (2) which is covered by a cover (3) of identical or similar textile material and is fixed to the textile material of the bandage (1) by means of borders projecting beyond the pad (2), characterised in that the cover (3) is provided on its side facing the pad (2) with an elastic, thermoplastic coating (6) which is stuck on to the textile material (1) of the bandage in the region of the borders (4, 5) by heating and the softening point of which is situated below that of the textile materials.

2. Bandage according to claim 1, characterised in that the plastic coating (6) consists of polyurethane.

3. Bandage according to claim 1 or claim 2, characterised in that the plastic coating (6) is painted on to the cover (3).

4. Bandage according to claim 1 or claim 2, characterised in that the plastic coating (6) is sprayed on to the cover (3).

## Revendications

1. Bandage (1), par exemple manchon, consistant en une matière textile élastique, pour le traitement d'articulations et muni d'un rembourrage (2) qui est recouvert d'un revêtement (3) en matière textile identique ou analogue et qui est fixé à la matière textile du bandage (1) au moyen de bords dépassant du rembourrage (2), caractérisé en ce que le revêtement (3) est muni, sur sa face tournée vers le rembourrage (2), d'une couche (6) de matière synthétique élastique et thermoplastique qui est collée par chauffage sur la matière textile (1) dans la zone des bords (4, 5) et dont la température de ramollissement est située au-dessous de celle des matières textiles.

2. Bandage selon la revendication 1, caractérisé en ce que la couche de matière synthétique (6) consiste en du polyuréthanne.

3. Bandage selon la revendication 1 ou 2, caractérisé en ce que la couche de matière synthétique (6) est étalée par application sur le revêtement (3).

4. Bandage selon la revendication 1 ou 2, caractérisé en ce que la couche de matière synthétique (6) est déposée par projection sur le revêtement (3).
